# EUROPÄISCHE PATENTANMELDUNG

(11) **EP 2 911 075 A1**
(43) Veröffentlichungstag der Anmeldung: **26.08.2015**
(21) Anmeldenummer: 14156479.9
(22) Anmeldetag: 25.02.2014
(51) Int. Cl.: G06F 19/00

(54) **System zur Bestimmung einer Eignung eines Wirkstoffs für die transdermale oder transmukosale Applikation sowie entsprechendes Verfahren**

(71) Anmelder: LTS LOHMANN Therapie-Systeme AG, 56626 Andernach (DE)
(72) Erfinder: Franke, Hanshermann, 22299 Hamburg (DE); Mohr, Patrick, 53489 Bad Breisig (DE); Eifler, René, 56072 Koblenz (DE); Bauer, Marius, 56626 Andernach (DE)
(74) Vertreter: Held, Stephan

(57) **Zusammenfassung**

Erfindungsgemäß soll eine Vorrichtung sowie ein Verfahren zur Bestimmung einer Eignung eines Wirkstoffs für die transdermale oder transmukosale Applikation angegeben werden. Diese Aufgabe wird durch ein System gelöst, das umfasst:
- mindestens eine Eingabeeinrichtung (101) zur Eingabe zumindest einer Bezeichnung eines Wirkstoffs;
- Datenbank (110) zum Speichern einer Vielzahl von Daten zumindest bezüglich des Wirkstoffs;
- Recheneinheit (102) zur Berechnung einer Eignung des Wirkstoffs für transdermale und/oder transmukosale Applikation unter Berücksichtigung von über die Eingabeeinrichtung (101) eingegebenen Daten und/oder der in der Datenbank (110) gespeicherten Daten;
- eine Ausgabeeinrichtung (103) zur Anzeige der berechneten Eignung.

## Beschreibung

Die Erfindung betrifft ein System zur Bestimmung einer Eignung eines Wirkstoffs für die transdermale und/oder transmukosale Applikation.

Die transdermale Applikation von Wirkstoffen ist bekannt. Das wohl bekannteste Beispiel ist das Nikotinpflaster. Dieses wird auf die Haut geklebt und setzt den Wirkstoff kontrolliert frei, welcher dann über die Haut resorbiert wird. Der Wirkstoff gelangt in das Blutgefäßsystem, ohne vorzeitig im Magen-Darm-Trakt oder der Leber abgebaut zu werden. Synonym wird auch der Begriff des transdermalen therapeutischen Systems verwendet.

Ebenso kennt man die Applikation bzw. Verabreichung von Wirkstoffen über die Schleimhäute (Mukosa). Ein Beispiel für die transmukosale Applikation ist die Lutschtablette, die oral eingenommen, aber nicht geschluckt, sondern im Mund belassen wird und sich dort auflösen soll. Transmukosale therapeutische Systeme werden dann bevorzugt eingesetzt, wenn das Wirkungsgebiet des Wirkstoffs im Mund-Rauchenraum liegt (z. B. Lokalanästhetika und Entzündungshemmer), oder der Wirkstoff direkt über die Mundschleimhaut in den Blutkreislauf gelangen soll. Letzteres hat dabei zwei Vorteile. Einerseits gelangt das Medikament sehr schnell in den Blutkreislauf, anderseits kann so das Pfortadersystem der Leber umgangen werden.

In der Forschung stellt es sich als besonderes Problem dar, vorherzusagen, ob ein bestimmter Wirkstoff transdermal und/oder transmukosal appliziert werden kann. Häufig sind aufwändige klinische Studien notwendig, um festzustellen, dass für einen bestimmten Anwendungsfall eine transdermale oder transmukosale Applikation nicht gegeben ist. Des Weiteren stellt es sich als Problem dar, bekannte Modelle, mit denen eine Vorhersage möglich ist, zu errechnen. Die maschinelle Verarbeitung entsprechender Modelle ist rechenintensiv und erfordert viel Speicher. Die Ergebnisse sind ungenau und schwierig zu klassifizieren.

Ausgehend von diesem Stand der Technik stellt sich die vorliegende Erfindung die Aufgabe, ein verbessertes System zur Bestimmung einer Eignung eines Wirkstoffs für die transdermale oder transmukosale Applikation bereitzustellen.

Diese Aufgabe wird erfindungsgemäß durch ein System gemäß dem Anspruch 1 sowie durch ein Verfahren gemäß dem Anspruch 10 gelöst.

I nsbesondere wird die Aufgabe durch ein System zur Bestimmung einer Eignung eines Wirkstoffs für die transdermale oder transmukosale Applikation gelöst, wobei das System umfasst:
- mindestens eine Eingabeeinrichtung zur Eingabe zumindest einer Bezeichnung eines Wirkstoffs;
- Datenbank zum Speichern einer Vielzahl von Daten zumindest bezüglich des Wirkstoffs;
- Recheneinheit zur Berechnung einer Eignung des Wirkstoffs für die transdermale oder transmukosale Applikation unter Berücksichtigung von über die Eingabeeinrichtung eingegebenen Daten und/oder der in der Datenbank gespeicherten Daten;
- eine Ausgabeeinrichtung zur Anzeige der berechneten Eignung.

Ein besonderer Vorteil dieses Systems besteht darin, dass die Eignung in einfacher Weise mittels einer Datenverarbeitungseinrichtung, nämlich der Recheneinheit, berechnet werden kann. Natürlich kann eine Vorhersage der Eignung eines bestimmten Wirkstoffs für die transdermale oder -mukosale Applikation die klinische Studie nicht ersetzen. Dennoch besteht ein Vorteil des erfindungsgemäßen Systems darin, Kandidaten die für eine entsprechende Applikation gänzlich ungeeignet sind, frühzeitig zu erkennen.

In einem Ausführungsbeispiel ist die Recheneinheit dazu ausgebildet, zur Berechnung der Eignung ein gewichtetes Mittel über eine Vielzahl von Kennzahlen zu bilden. Vorzugsweise führen die angegebenen und/oder gespeicherten Daten zu Kennzahlen, die in einzelnen Themengebieten die Eignung des Wirkstoffs für die transdermale oder transmukosale Applikation angeben. Erfindungsgemäß soll eine insbesondere gewichtete Mittelung über die Vielzahl von Kennzahlen stattfinden. Beispielsweise können mindestens vier oder sechs oder acht oder zehn Kennzahlen zu einem Gesamtergebnis gemittelt werden, um die Eignung eines bestimmten Wirkstoffs für die transdermale oder transmukosale Applikation anzugeben. Diese Vielzahl von Parametern führt zu einem robusten und aussagekräftigen Ergebnis.

Die Recheneinheit kann dazu ausgebildet sein, die/eine Kennzahl unter Berücksichtigung jeweils einer der folgenden Angaben zu ermitteln:
- mindestens eine Angabe zur Strukturformel des Wirkstoffs, insbesondere des molekulares Gewichts;
- mindestens eine Angabe über die Säurestärke des Wirkstoffs, insbesondere den pk_{A}-Wert oder pk_{S}-Wert
- mindestens eine Angabe zum Schmelzpunkt des Wirkstoffs;
- mindestens eine Angabe zur Halbwertszeit;
- mindestens eine Angabe zur Wasserlöslichkeit und/oder Fettlöslichkeit des Wirkstoffs, insbesondere den Log P-Wert;
- mindestens eine Angabe zur Hautdurchlässigkeit und/oder zur in vitro Permeabilität (z.B. Permeabilitätsrate);
- mindestens eine Angabe zur (oralen) Bioverfügbarkeit;
- mindestens eine Angabe zur (oralen) Dosis, insbesondere (oralen) Tagesdosis;
- mindestens eine Angabe bezüglich des Metabolismus;
- mindestens eine Angabe bezüglich des Hautreizungspotentials;
- mindestens eine Angabe bezüglich der Indikation; und/oder
- mindestens eine Angabe bezüglich der Dosis-Wirkungs-Beziehung.

Diese Angaben sind für die transdermale, aber im Wesentlichen auch für die transmukosale Applikation besonders aussagekräftig und lassen sich einfach und effizient von der Recheneinheit verarbeiten.

Zumindest einige der Kennzahlen können eine Auswahl von Zahlen aus der Menge der ganzen Zahlen sein. I m Endeffekt ist es möglich, die angegebenen Angaben, beispielsweise bezüglich der Strukturformel, auf einen Indexwert bzw. eine Kennzahl abzubilden. Bevorzugt kann diese Kennzahl ganzzahlig sein, so dass einfache und aussagekräftige Ergebnisse entstehen. In einem weiteren bevorzugten Ausführungsbeispiel handelt es sich bei den Kennzahlen um eine Auswahl von ganzzahligen Zahlen innerhalb eines vorgegebenen Intervalls. Vorzugsweise hat diese Menge eine relativ geringe Kardinalität. Beispielsweise können in dieser Menge weniger als 15 Elemente oder weniger als 10 Elemente oder weniger als 7 Elemente enthalten sein. In einer Ausführungsform umfasst die Menge die folgenden (Kenn-) Zahlen {-3, -2, -1, 0, 1, 2, 3}, in einer weiteren Ausführungsform die (Kenn-) Zahlen {-5, -4, -3, -2, -1, 0, 1, 2, 3, 4, 5}, oder aber auch {0, 1, 2, 3, 4, 5, 6, 7, 8, 9, 10}. Ebenso können Kommazahlen, beispielsweise mit einer Genauigkeit von einer Nachkommerstelle als Kennzahlen verwendet werden.

Die Recheneinheit kann dazu ausgebildet sein, bei der Berechnung der Eignung des Wirkstoffs
- die mindestens eine Angabe oder Kennzahl zur Strukturformel des Wirkstoffs; und/oder
- die mindestens eine Angabe oder Kennzahl zur Bioverfügbarkeit; und/oder
- die mindestens eine Angabe oder Kennzahl zur Dosis mit mindestens je 5% und maximal 40%, insbesondere mit mindestens je 10% und maximal 30%, zu berücksichtigen.

Wie bereits erläutert, wird vorzugsweise eine gewichtete Mittelung über die Kennzahlen vorgenommen. Die Strukturformel und/oder die Bioverfügbarkeit bzw. die Dosis können besonders stark bei dem Ergebnis bezüglich der transdermalen und/oder transmukosalen Eignung berücksichtigt werden. In einem Ausführungsbeispiel können die zugehörigen Kennzahlen bis maximal 30% des Ergebnisses beeinflussen. Vorzugsweise findet die Strukturformel und die Bioverfügbarkeit bzw. Dosis mit mindestens 5% Einzug in das Gesamtergebnis.

Die Recheneinheit kann dazu ausgebildet sein, bei der Berechnung der Eignung des Wirkstoffs
- die mindestens eine Angabe oder Kennzahl zur Säurestärke des Wirkstoffs; und/oder
- die mindestens eine Angabe oder Kennzahl zum Schmelzpunkt des Wirkstoffs; und/oder
- die mindestens eine Angabe oder Kennzahl zur Wasserlöslichkeit und/oder Fettlöslichkeit des Wirkstoffs; und/oder
- die mindestens eine Angabe oder Kennzahl zur Hautdurchlässigkeit; und/oder
- die mindestens eine Angabe bezüglich des Hautreizungspotentials
mit mindestens je 2% und maximal 20%, insbesondere mit mindestens je 5% und maximal 15% zu berücksichtigen.

Die genannten Angaben sind für das Gesamtergebnis wichtig. Jedoch kann es vorteilhaft sein, diese mit einer geringeren Gewichtung zu berücksichtigen. In einem bevorzugten Ausführungsbeispiel liegt diese Gewichtung innerhalb des Bereichs von 3-10% pro Kennzahl. Diese Gewichtung kann zu einer besonders effizienten Berechnung führen.

In einem Ausführungsbeispiel kann die Datenbank Abbildungstabellen zur Abbildung einzelner Angaben auf Kennzahlen umfassen. Beispielsweise können die Abbildungstabellen Intervalle angeben, wann eine bestimmte Angabe einer bestimmten Kennzahl zugeordnet wird. Es können Abbildungstabellen zur Abbildung der
- mindestens einen Angabe zur Strukturformel des Wirkstoffs, insbesondere des molekularen Gewichts;
- mindestens einen Angabe über die Säurestärke des Wirkstoffs, insbesondere des pk_{A}-Werts oder pk_{S}-Werts
- mindestens einen Angabe zum Schmelzpunkt des Wirkstoffs;
   und/oder
- mindestens einen Angabe zur biologischen Halbwertszeit; und/oder
- mindestens einen Angabe zur Wasserlöslichkeit und/oder Fettlöslichkeit des Wirkstoffs, insbesondere den Log P-Wert; und/oder
- mindestens einen Angabe zur Dosis
auf Kennzahlen in der Datenbank gespeichert sein.

In einem weiteren Ausführungsbeispiel gibt es eine einzige Abbildungstabelle, die für mehrere Angaben Abbildungen auf entsprechende Kennzahlen bereitstellt.

Das System kann mindestens eine Messeinrichtung zur Bestimmung des molekularen Gewichts und/oder der Säurestärke und/oder der Wasserlöslichkeit und/oder des Schmelzpunkts umfassen. Vorzugsweise kann das System also einige der Parameter selbstständig bestimmen und bei der Ermittlung der Eignung des Wirkstoffs für die transdermale oder transmukosale Applikation berücksichtigen.

Die genannte Aufgabe wird des Weiteren durch ein Verfahren zur Bestimmung einer Eignung eines Wirkstoffs gemäß Anspruch 9 gelöst.

I nsbesondere wird die Aufgabe durch ein Verfahren zur Bestimmung einer Eignung eines Wirkstoffs für die transdermale oder transmukosale Applikation gelöst, wobei das Verfahren die folgenden Schritte umfasst:
- Erfassen von Kenndaten des Wirkstoffs;
- Ermitteln von Kennzahlen jeweils für die Kenndaten des Wirkstoffs;
- Errechnen eines gewichteten Mittels übe die Kennzahlen;
- Anzeigen einer berechneten Eignung des Wirkstoffes unter Verwendung des gewichteten Mittels.

Vorzugsweise werden diese Schritte von dem bereits beschriebenen System ausgeführt. Es ergeben sich ähnliche Vorteile, wie diese bereits im Zusammenhang mit der Vorrichtung beschrieben wurden.

Die bei dem Verfahren berücksichtigten Daten können umfassen:
- mindestens eine Angabe zur Strukturformel des Wirkstoffs, insbesondere des molekulares Gewichts;
- mindestens eine Angabe über die Säurestärke des Wirkstoffs, insbesondere den pk_{A}-Wert oder pk_{S}-Wert
- mindestens eine Angabe zum Schmelzpunkt des Wirkstoffs;
- mindestens eine Angabe zur Halbwertszeit;
- mindestens eine Angabe zur Wasserlöslichkeit und/oder Fettlöslichkeit des Wirkstoffs, insbesondere den Log P-Wert;
- mindestens eine Angabe zur Hautdurchlässigkeit;
- mindestens eine Angabe zur Bioverfügbarkeit;
- mindestens eine Angabe zur Dosis, insbesondere Tagesdosis;
- mindestens eine Angabe bezüglich des Metabolismus;
- mindestens eine Angabe bezüglich des Hautreizungspotentials;
- mindestens eine Angabe bezüglich der Indikation; und/oder
- mindestens eine Angabe bezüglich der Dosis-Wirkungs-Beziehung.

Vorzugsweise umfasst das Verfahren einen Schritt des Auslesens mindestens einer Kennzahl aus einer/der Datenbank. Diese Kennzahl kann unter Berücksichtigung der besagten Angaben ermittelt werden. Die Datenbank stellt I ntervalle bereit, um die Angaben auf bestimmte Kennzahlen abzubilden.

Das Verfahren kann einen Schritt des Vergleichens der gewichteten Mittel mit einem Schwellenwert umfassen. Vorzugsweise führt dieser Vergleich dazu, dass ein bestimmter Wirkstoff als für die transdermale oder transmukosale Applikation geeignet oder nicht geeignet angesehen wird. In einem Ausführungsbeispiel gilt ein bestimmter Wirkstoff als für die transdermale oder transmukosale Applikation geeignet, wenn das gewichtete Mittel größer als ein Schwellenwert, insbesondere größer als 0 oder 1 ist.

Die Aufgabe kann des Weiteren durch ein computerlesbares Medium mit Instruktionen zur I mplementierung einer der beschriebenen Verfahren gelöst werden. Die I nstruktionen sind dabei dafür gedacht, auf einem Computer ausgeführt zu werden.

Weitere vorteilhafte Ausführungsformen ergeben sich anhand der Unteransprüche.

Nachfolgend wird die Erfindung mittels mehrerer Ausführungsbeispiele beschrieben, die anhand von Abbildungen näher erläutert werden. Hierbei zeigen:
- Fig. 1: ein System zur Bestimmung einer Eignung eines Wirkstoffs für die transdermale Applikation;
- Fig. 2: eine Abbildungstabelle zur Abbildung eines Parameters des Wirkstoffs auf jeweils eine Kennzahl.

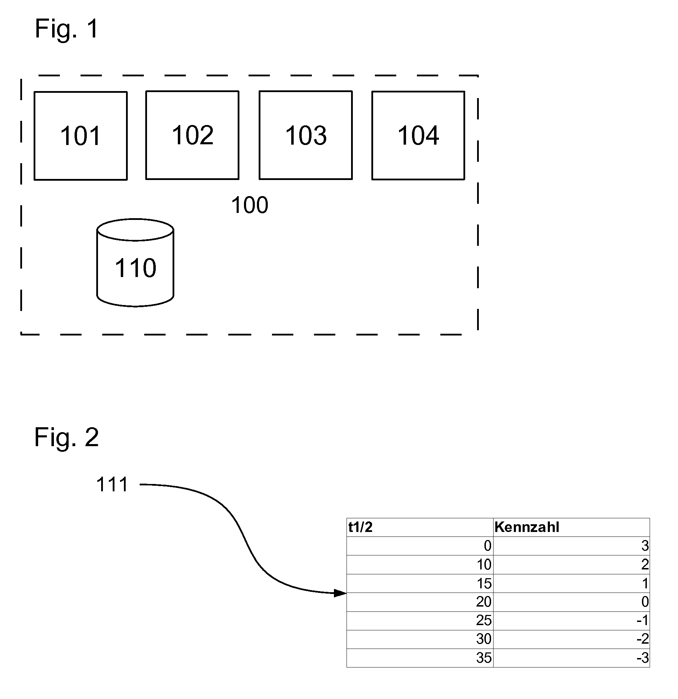

Fig. 1 zeigt ein System 100 zur Bestimmung einer Eignung eines Wirkstoffs für die transdermale Applikation. Das System umfasst eine Eingabeeinrichtung 101 zur Eingabe zumindest einer Bezeichnung eines Wirkstoffs. Die Eingabeeinrichtung 101 kann des Weiteren dazu geeignet sein, Angaben zur Strukturformel des Wirkstoffs, der Säurestärke, dem Schmelzpunkt, der Halbwertszeit, der Wasserlöslichkeit, der Fettlöslichkeit, der Bioverfügbarkeit, der Dosis, insbesondere Tagesdosis, des Metabolismus, der Angabe bezüglich des Hautreizungspotentials und/oder der Dosis-Wirkungsbeziehung zu empfangen. Das System 100 weist des Weiteren eine Recheneinheit 102 auf, die die eingegebenen Daten und weitere Daten, die beispielsweise in einer Datenbank 110 gespeichert sind, verarbeitet, um die Eignung des Wirkstoffs für die transdermale Applikation zu bestimmen.

Eine Ausgabeeinrichtung 103 des Systems 100 kann die Ergebnisse anzeigen oder einen Benutzer auffordern, Eingaben zu tätigen. Beispielsweise kann der Benutzer aufgefordert werden, Angaben bezüglich der oben genannten Parameter zu machen.

In einem Ausführungsbeispiel sind einige dieser Angaben bereits in der Datenbank 110 gespeichert und können beispielsweise durch die Eingabe der Bezeichnung des Wirkstoffs abgerufen werden.

Optional weist das System 100 eine Messeinrichtung 104 auf, das zumindest einige der bereits genannten Angaben erfasst und zur Weiterverarbeitung durch die Recheneinheit 102 bereitstellen kann. Beispielsweise kann es sich hierbei um eine Einrichtung zur Ermittlung des Schmelzpunktes, eine Spektralanalyseeinrichtung und ähnliche Vorrichtungen handeln.

Nach einer Erfassung der Angaben durch die Recheneinheit 102 werden diese in einem Ausführungsbeispiel auf Kennzahlen abgebildet. Eine entsprechende Abbildung kann durch eine Rechenvorschrift erfolgen.

In einem Ausführungsbeispiel nutzt die Recheneinheit 102 die Datenbank 110, um eine geeignete Abbildung vorzunehmen. Beispielsweise kann eine Angabe bezüglich der Halbwertszeit verwendet werden, um aus einer in der Datenbank 110 gespeicherten Halbwertszeit-Abbildungstabelle 111 eine zugeordnete Kennzahl zu ermitteln.

Strukturell ist die Halbwertszeit-Abbildungstabelle 111 so aufgebaut, dass sie zwei Spalten aufweist, wobei die erste Spalte den Parameterwert der Halbwertszeit "T1/2" und die zweite Spalte die "Kennzahl" angibt. Neben der Überschriftszeile umfasst die Halbwertszeit-Abbildungstabelle 111 sieben weitere Zeilen, die dazu geeignet sind, I ntervalle für Parameter zu definieren und diesen eine bestimmte Kennzahl zuzuordnen. In dem gezeigten Ausführungsbeispiel ist die Halbwertszeit-Abbildungstabelle 111 derart zu interpretieren, dass dem Intervall 0 bis kleiner 10 h (= Stunden) die Kennzahl 3, dem Intervall 10 bis kleiner 15 h die Kennzahl 2, dem Intervall 15 bis kleiner 20 h die Kennzahl 1, dem Intervall 20 bis kleiner 25 h die Kennzahl 0, dem Intervall 25 bis kleiner 30 h die Kennzahl -1, dem Intervall 30 bis kleiner 35 h die Kennzahl -2 und dem Intervall größer 35 h die Kennzahl -3 zugeordnet ist.

Hat ein Wirkstoff beispielsweise eine Halbwertszeit von 9 h, so würde ihm die Recheneinheit 102 mittels der Halbwertszeit-Abbildungstabelle 111 die Kennzahl 3 zuordnen.

Die Recheneinheit ist dazu ausgebildet, für jeden vorgegebenen Parameter bzw. für jede Angabe zu dem Wirkstoff eine Kennzahl zu ermitteln und diese Kennzahlen nach einer Gewichtungsvorschrift zu mitteln. Beispielsweise kann die Kennzahl für die Strukturformel mit ca. 20% und die Tagesdosis mit ca. 15% eine prominente Rolle bei der Berechnung des Gesamtergebnisses einnehmen. Mit einer etwas geringeren Gewichtung (beispielsweise ca. 10%) kann eine Kennzahl bezüglich der Säurestärke des Wirkstoffs, eines Schmelzpunkts, einer Wasserlöslichkeit und einer Gefahr zur Bildung von Hautirritationen (Hautreizungspotential) berücksichtigt werden. Mit einer noch geringeren Gewichtung (beispielsweise 5%) können Kennzahlen, die die Halbwertszeit, die Wasserlöslichkeit und den Metabolismus angeben, berücksichtigt werden.

Wendet man beispielsweise das erfindungsgemäße Verfahren auf den Wirkstoff Nikotin an, so ergeben sich die folgenden Ergebnisse. Nikotin hat ein molekulares Gewicht von 62 g/mol, der Schmelzpunkt liegt bei -79°C und der pKA-Wert ist 8,9. Die Halbwertszeit von Nikotin liegt zwischen 1 und 6 h, wobei ein log P-Wert von 1,17 berechnet wurde. Die notwendige Tagesdosis liegt üblicherweise zwischen 7 und 21 mg, wobei man bei einer oralen Verabreichung von einem Wirkungsgrad von 20-45% ausgeht.

Für Nikotin ergeben sich gemäß dem erfindungsgemäßen Verfahren die folgenden Kennzahlen:
- Strukturformel = 4;
- pKA-Wert = 2;
- Schmelzpunkt, Halbwertszeit, log P-Wert = 6;
- Hautdurchlässigkeit = 4;
- Tagesdosis, Wasserlöslichkeit = -2;
- PD-Daten = 2;
- Metabolismus und Wahrscheinlichkeit für Hautirritationen = 0.

Insgesamt ergibt sich daher ein gewichtetes Mittel von ca. 2,4. Das gewichtete Mittel ist somit positiv, was angibt, dass ein bestimmter Wirkstoff, im Beispiel Nikotin, für die transdermale Applikation geeignet ist. Ein gewichtetes Mittel größer als der Schwellenwert 2 zeigt in diesem Ausführungsbeispiel, dass hier ein sehr gut geeigneter Wirkstoff vorliegt.

Demgegenüber führt das erfindungsgemäße Verfahren bei einer Bewertung des Wirkstoffs Enalapril zu einem gewichteten Mittel von ca. -1. Das negative Ergebnis zeigt an, dass dieser Wirkstoff für eine transdermale Applikation eher schlecht geeignet ist.

Das beschriebene Verfahren wird teilweise oder vollständig von der Recheneinheit 102 implementiert.

Das beschriebene System kann ebenso für die Feststellung der Eignung eines bestimmten Wirkstoffs für die transmukosale Applikation eingesetzt werden.

### Bezugszeichen

- 100: System
- 101: Eingabeeinrichtung
- 102: Recheneinheit
- 103: Ausgabeeinrichtung
- 104: Messeinrichtung
- 110: Datenbank
- 111: Halbwertszeit-Abbildungstabelle

## Patentansprüche

1. System zur Bestimmung einer Eignung eines Wirkstoffs für die transdermale oder transmukosale Applikation, umfassend:
- mindestens eine Eingabeeinrichtung (101) zur Eingabe zumindest einer Bezeichnung eines Wirkstoffs;
- Datenbank (110) zum Speichern einer Vielzahl von Daten zumindest bezüglich des Wirkstoffs;
- Recheneinheit (102) zur Berechnung einer Eignung des Wirkstoffs für die transdermale oder transmukosale Applikation unter Berücksichtigung von über die Eingabeeinrichtung (101) eingegebenen Daten und/oder der in der Datenbank (110) gespeicherten Daten;
- eine Ausgabeeinrichtung (103) zur Anzeige der berechneten Eignung.

2. System nach Anspruch 1
**dadurch gekennzeichnet, dass**
die Recheneinheit (102) dazu ausgebildet ist, zur Berechnung der Eignung ein gewichtetes Mittel über eine Vielzahl, insbesondere mindestens 4 oder mindestens 6 oder mindestens 8 oder mindestens 10, Kennzahlen zu bilden.

3. System nach einem der vorhergehenden Ansprüche,
**dadurch gekennzeichnet, dass**
die Recheneinheit (102) dazu ausgebildet ist, Kennzahlen unter Berücksichtigung jeweils einer der folgenden Angaben zu ermitteln:
- mindestens eine Angabe zur Strukturformel des Wirkstoffs, insbesondere molekulares Gewicht;
- mindestens eine Angabe über die Säurestärke des Wirkstoffs, insbesondere den pk_{A}-Wert oder pk_{S}-Wert
- mindestens eine Angabe zum Schmelzpunkt des Wirkstoffs;
- mindestens eine Angabe zur Halbwertszeit;
- mindestens eine Angabe zur Wasserlöslichkeit und/oder Fettlöslichkeit des Wirkstoffs, insbesondere den Log P-Wert;
- mindestens eine Angabe zur Hautdurchlässigkeit;
- mindestens eine Angabe zur Bioverfügbarkeit;
- mindestens eine Angabe zur Dosis, insbesondere Tagesdosis;
- mindestens eine Angabe bezüglich des Metabolismus;
- mindestens eine Angabe bezüglich des Hautreizungspotentials;
- mindestens eine Angabe bezüglich der I ndikation; und/oder
- mindestens eine Angabe von Daten zur Beschreibung der Dosis-Wirkungs-Beziehung.

4. System nach einem der vorhergehenden Ansprüche,
**dadurch gekennzeichnet, dass**
zumindest einige, vorzugsweise alle, der Kennzahlen eine Auswahl von Zahlen aus der Menge der ganzen Zahlen oder von Zahlen mit einer Dezimale, insbesondere innerhalb eines vorgegebenen intervalls, sind.

5. System nach einem der vorhergehenden Ansprüche, insbesondere nach Anspruch 3,
**dadurch gekennzeichnet, dass**
die Recheneinheit (102) dazu ausgebildet ist, bei der Berechnung der Eignung des Wirkstoffs
- die mindestens eine Angabe oder Kennzahl zur Strukturformel des Wirkstoffs; und/oder
- die mindestens eine Angabe oder Kennzahl zur Bioverfügbarkeit;
und/oder
- die mindestens eine Angabe oder Kennzahl zur Dosis mit mindestens 5% und maximal 40%, insbesondere mit mindestens 10% und maximal 30%, zu berücksichtigen.

6. System nach einem der vorhergehenden Ansprüche,
**dadurch gekennzeichnet, dass** die Recheneinheit (102) dazu ausgebildet ist, bei der Berechnung der Eignung des Wirkstoffs
- die mindestens eine Angabe oder Kennzahl zur Säurestärke des Wirkstoffs; und/oder
- die mindestens eine Angabe oder Kennzahl zum Schmelzpunkt des Wirkstoffs; und/oder
- die mindestens eine Angabe oder Kennzahl zur Wasserlöslichkeit und/oder Fettlöslichkeit des Wirkstoffs; und/oder
- die mindestens eine Angabe oder Kennzahl zur Hautdurchlässigkeit;
und/oder
- die mindestens eine Angabe bezüglich des Hautreizungspotentials
mit mindestens 2% und maximal 20%, insbesondere mit mindestens 5% und maximal 15%, zu berücksichtigen.

7. System nach einem der vorhergehenden Ansprüche,
**dadurch** h **gekennzeichnet,** dass die Datenbank (110) Abbildungstabellen (111) zur Abbildung der
- mindestens einen Angabe zur Strukturformel des Wirkstoffs, insbesondere des molekularen Gewichts;
- mindestens einen Angabe über die Säurestärke des Wirkstoffs, insbesondere des pk_{A}-Werts oder pk_{S}-Werts
- mindestens einen Angabe zum Schmelzpunkt des Wirkstoffs;
und/oder
- mindestens einen Angabe zur biologischen Halbwertszeit; und/oder
- mindestens einen Angabe zur Wasserlöslichkeit und/oder Fettlöslichkeit des Wirkstoffs, insbesondere den Log P-Wert; und/oder
- mindestens einen Angabe zur Dosis
auf jeweils mindestens eine Kennzahl speichert.

8. System nach einem der vorhergehenden Ansprüche,
**gekennzeichnet durch**
- mindestens eine Messeinrichtung zur Bestimmung des molekularen Gewichts und/oder der Säurestärke und/oder der Wasserlöslichkeit und/oder des Schmelzpunkts.

9. Verfahren zur Bestimmung einer Eignung eines Wirkstoffs für die transdermale oder transmukosale Applikation, insbesondere mittels des Systems (100) gemäß einem der vorhergehenden Ansprüche, umfassend die Schritte:
- Erfassen von Kenndaten des Wirkstoffs;
- Ermitteln von Kennzahlen jeweils für die Kenndaten des Wirkstoffs;
- Errechnen eines gewichteten Mittels übe die Kennzahlen;
- Anzeigen einer berechneten Eignung des Wirkstoffes unter Verwendung des gewichteten Mittels.

10. Verfahren nach Anspruch 9,
**dadurch gekennzeichnet, dass** die Kenndaten umfassen:
- mindestens eine Angabe zur Strukturformel des Wirkstoffs, insbesondere des molekulares Gewichts;
- mindestens eine Angabe über die Säurestärke des Wirkstoffs, insbesondere den pk_{A}-Wert oder pk_{S}-Wert
- mindestens eine Angabe zum Schmelzpunkt des Wirkstoffs;
- mindestens eine Angabe zur Halbwertszeit;
- mindestens eine Angabe zur Wasserlöslichkeit und/oder Fettlöslichkeit des Wirkstoffs, insbesondere den Log P-Wert;
- mindestens eine Angabe zur Hautdurchlässigkeit;
- mindestens eine Angabe zur Bioverfügbarkeit;
- mindestens eine Angabe zur Dosis, insbesondere Tagesdosis;
- mindestens eine Angabe bezüglich des Metabolismus;
- mindestens eine bezüglich des Hautreizungspotentials;
- mindestens eine Angabe bezüglich der Indikation; und/oder
- mindestens eine Angabe von Daten zur Beschreibung der Dosis-Wirkungs-Beziehung.

11. Verfahren nach Anspruch 9 oder 10,
**gekennzeichnet durch**
ein Ermitteln mindestens eines Teils der Kenndaten mittels mindestens einer Messeinrichtung.

12. Verfahren nach einem der Ansprüche 9 bis 11,
**gekennzeichnet durch**
ein Auslesen mindestens einer Kennzahl aus einer/der Datenbank (110), die
- mindestens einer Angabe zur Strukturformel des Wirkstoffs, insbesondere des molekularen Gewichts;
- mindestens einer Angabe über die Säurestärke des Wirkstoffs, insbesondere dem pk_{A}-Wert oder pk_{S}-Wert;
- mindestens einer Angabe zum Schmelzpunkt des Wirkstoffs;
und/oder
- mindestens einer Angabe zur biologischen Halbwertszeit; und/oder
- mindestens einer Angabe zur Wasserlöslichkeit und/oder Fettlöslichkeit des Wirkstoffs, insbesondere dem Log P-Wert; und/oder
- mindestens einer Angabe zur Dosis
zugeordnet ist.

13. Verfahren nach einem der Ansprüche 9 bis 12,
**gekennzeichnet durch**
ein Vergleichen des gewichteten Mittels mit einem Schwellenwert, wobei der Wirkstoff als für die transdermale oder transmukosale Applikation geeignet gilt und angezeigt wird, wenn das gewichtete Mittel größer als der Schwellenwert ist.

14. Computerlesbares Medium mit Instruktionen zur Implementierung des Verfahrens nach einem der Ansprüche 9 bis 13, wenn die I nstruktionen auf einem Computer ausgeführt werden.
